# EUROPEAN PATENT SPECIFICATION

(11) **EP 3 367 100 B1**
(45) Date of publication and mention of the grant of the patent: **18.12.2019**
(21) Application number: 18158737.9
(22) Date of filing: 27.02.2018
(51) Int. Cl.: G01N 33/574

(54) **METHOD FOR ASSISTING DETERMINATION OF INVASIVENESS OF MYXOFIBROSARCOMA, AND USE OF A KIT IN THE METHOD**
VERFAHREN ZUR UNTERSTÜTZUNG DER BESTIMMUNG DER INVASIVITÄT EINES MYXOFIBROSARKOMS UND VERWENDUNG EINES KITS IM VERFARHEN
PROCÉDÉ D'AIDE À LA DÉTERMINATION DU CARACTÈRE INVASIF DU MYXOFIBROSARCOME ET UTILISATION D'UN KIT DANS LE PROCÉDÉ

(30) Priority: 27.02.2017 JP 2017035001
(43) Date of publication of application: 29.08.2018
(73) Proprietor: National Cancer Center, Tokyo 1040045 (JP); Keio University, Tokyo 108-8345 (JP); SYSMEX CORPORATION, Kobe-shi Hyogo 651-0073 (JP)
(72) Inventor: Kikuta, Kazutaka, Tokyo, 1608582 (JP); Nakamura, Masaya, Tokyo, 1608582 (JP); Kawai, Akira, Tokyo, 1040045 (JP); Kondo, Tadashi, Tokyo, 1040045 (JP)
(74) Representative: De Clercq & Partners

(56) References cited:
- SHA LOU ET AL: "High-grade sarcoma diagnosis and prognosis: Biomarker discovery by mass spectrometry imaging", PROTEOMICS, vol. 16, no. 11-12, 1 June 2016 (2016-06-01), pages 1802-1813, XP055469390, DE ISSN: 1615-9853, DOI: 10.1002/pmic.201500514
- KIM M ET AL: "Epigenetic down-regulation and suppressive role of DCBLD2 in gastric cancer cell proliferation and invasion", MOLECULAR CANCER RESE, AMERICAN ASSOCIATION FOR CANCER RESEARCH, US, vol. 6, no. 2, 1 February 2008 (2008-02-01), pages 222-230, XP008135573, ISSN: 1541-7786, DOI: 10.1158/1541-7786.MCR-07-0142
- KIKUTA KAZUTAKA ET AL: "Discoidin, CUB and LCCL domain-containing protein 2 (DCBLD2) is a novel biomarker of myxofibrosarcoma invasion identified by global protein expression profiling", BIOCHIMICA ET BIOPHYSICA ACTA (BBA) - PROTEINS & PROTEOMICS, ELSEVIER, NETHERLANDS, vol. 1865, no. 9, 29 June 2017 (2017-06-29), pages 1160-1166, XP085143908, ISSN: 1570-9639, DOI: 10.1016/J.BBAPAP.2017.06.023

## Description

### TECHNICAL FIELD

The present invention relates to a method for assisting determination of the invasiveness of myxofibrosarcoma. The present invention also relates to the use of a kit in the method.

### BACKGROUND

DCBLD2, also called CLCP1, is known to be highly expressed in several malignant tumors. For example, E. Topkas, et. Al., European Journal of Cancer, Volume 50, Supplement 5, Page S60 describes that DCBLD2 can be a marker for osteosarcoma. In addition, Koshikawa K, et al., Oncogene. 2002 Apr 25; 21(18): 2822-8 describes that CLCP1 is highly expressed in metastatic lung cancer. In addition, US 2011/0293618 A1 describes a CLCP1 antibody for inhibiting cancer cell migration, invasion into the extracellular matrix/basement membrane, proliferation, metastasis and cytotoxicity activity in lung cancer and the like, and describes that the CLCP1 molecule is abundantly expressed in metastatic lung cancer.

Lou et al., Proteomics, 2016 Jun,16(11-12):1802-13 is a prior art document that discloses prognostic markers for sarcomas including myxofibrosarcoma but not DCBLD2.

### SUMMARY OF THE INVENTION

### TECHNICAL PROBLEMS

However, no literature describes the relationship between DCBLD2 and invasiveness of myxofibrosarcoma.

The effect of radiation therapy and anticancer drug therapy is limited on myxofibrosarcoma. Therefore, tumor resection is an established therapy, but the local recurrence rate is high and reaches as high as 50 to 60%. Extensive resection that encapsulates normal tissue around the tumor tissue is necessary, in order to enhance the radicality of resection. Invasiveness of the tumor affects the local recurrence rate and also influences the setting of the resection range, so it is important to accurately determine whether it is invasive or non-invasive.

In the resection of myxofibrosarcoma, the range of the tumor may actually be wider than a preoperative prediction based on the image. When it is determined to be an inappropriate resection edge in histological resection edge evaluation after surgery, additional extensive resection or postoperative radiotherapy may be required. On the other hand, in order to secure the postoperative function, it is not desirable to expand the resection range more than necessary. Therefore, even before surgery, it is desirable to be able to predict whether or not the invasion has reached the range that is drawn in the image.

### SOLUTIONS TO PROBLEMS

The present inventors have found that invasiveness of myxofibrosarcoma can be determined based on the expression level of DCBLD2 since the expression of DCBLD2 is particularly promoted in tissues in which invasiveness is observed in myxofibrosarcoma and have found that its specificity and sensitivity are high.

Accordingly, according to the present invention, there is provided a method for assisting determination of the invasiveness of myxofibrosarcoma in a patient, comprising: measuring the expression level of Discoidin, CUB and LCCL domain-containing protein 2 (DCBLD2) in a sample of affected tissue of said patient; and comparing said expression level of DCBLD2 measured in said sample of affected tissue of said patient with a predetermined threshold value, wherein said myxofibrosarcoma is determined to be invasive when said expression level of DCBLD2 is greater than or equal to a predetermined threshold value. In particular embodiments of the methods provided herein, in the determining step, when the expression level of DCBLD2 is less than the predetermined threshold value, myxofibrosarcoma of the patient is determined to be non-invasive. In particular embodiments, the expression level of DCBLD2 is measured by immunohistochemical staining using an anti-DCBLD2 antibody. In particular embodiments, the affected tissue sample of the patient comprises tumor cells.

Further, according to the present invention, there is provided use of a kit for determining the invasiveness of myxofibrosarcoma comprising a reagent capable of measuring the expression level of DCBLD2 in the determination assisting method.

### ADVANTAGEOUS EFFECTS OF INVENTION

The present invention assists in the determination of the invasiveness of myxofibrosarcoma.

### BRIEF DESCRIPTION OF THE DRAWINGS

Figs. 1A and 1B are MRI images of tumor tissues of myxofibrosarcoma. Fig. 1A is an invasive example, and a plurality of tail like patterns extends from the central tumor mass (arrow parts). Fig. 1B is a non-invasive example, and clear boundary is found in the central tumor mass.
Fig. 2 is a heat map of a protein group satisfying the criterion that the difference in mean values is twice or more and p value < 0.01 by Wilcoxon test, in the comparison between invasive group and non-invasive group.
Figs. 3A, 3B, 3C and 3D are photomicrographs (3A: G3, 3B: G2, 3C: G1, 3D: G0) of samples determined to be any one of G0 to G3 based on the staining intensity standard, as a result of immunohistochemical staining.
Fig. 4 is a schematic diagram showing an example of a determination apparatus for determining the invasiveness of myxofibrosarcoma of a subject.
Fig. 5 is a block diagram showing a functional configuration of the determination apparatus in Fig. 4.
Fig. 6 is a block diagram showing a hardware configuration of the determination apparatus shown in Fig. 4.
Fig. 7 is a flowchart for determining the invasiveness of myxofibrosarcoma, using the determination apparatus shown in Fig. 4.

### DETAILED DESCRIPTION OF THE PREFERRED EMBODIMENTS

In the method for assisting determination of the invasiveness of myxofibrosarcoma of the present invention, the expression level of DCBLD2 in a sample of affected tissue of a patient with myxofibrosarcoma is measured. The affected tissue contains a tumor tissue, a reaction layer around it, and a tissue suspected of being a tumor. The reaction layer contains macroscopic discoloration parts such as hemorrhagic foci, discolored muscles, and edematous tissues. Whether it corresponds to an affected tissue can be determined based on images such as simple X-ray, ultrasonic examination, CT, MRI, and PET. DCBLD2 is a protein encoded by the DCBLD2 gene, whose amino acid sequence is known. The amino acid sequence can be exemplified by those of Swiss-Plot database Accession No. Q96PD2. Myxofibrosarcoma is known to have a high local recurrence rate. Local recurrence, unlike recurrence with metastasis, refers to the appearance of tumor of the same tissue type near the original tumor after tumor resection. As used herein, the term "invasiveness" refers to local invasiveness associated with local recurrence.

The method of measuring the expression level of DCBLD2 in the affected tissue is not particularly limited as long as it is a method capable of quantitatively evaluating DCBLD2 expression level in a biological sample. For example, the expression level of DCBLD2 can be measured by immunohistochemical staining such as fluorescent antibody method, enzyme antibody method, heavy metal-labeled antibody method or radioisotope-labeled antibody method, Western blotting, two-dimensional fluorescence difference gel electrophoresis, enzyme immunoassay (ELISA), dot-blotting method or the like. The expression level of DCBLD2 may be the mRNA expression level of DCBLD2. Examples of methods of measuring the mRNA expression level include RT-PCR, Northern blotting, Branched DNA assay, in situ hybridization, and the like.

Here, the "expression level of DCBLD2" used in the present embodiment may be a value directly representing the expression level such as the number of moles of DCBLD2 protein or the number of copies of mRNA, or may be a labeled amount representing these values. Specifically, as the labeled amount, the expression level of DCBLD2 protein can be expressed by fluorescence intensity, wave height intensity or the like, and DCBLD2 mRNA can be expressed by fluorescence intensity, the number of PCR cycles until the fluorescence intensity reaches a certain value, amplification reaction time or the like.

In measuring the expression level of DCBLD2, a sample can be prepared from the affected tissue collected from a patient with myxofibrosarcoma by biopsy or surgery. Preferably, the affected tissue contains tumor cells. For example, a formalin-fixed paraffin-embedded (FFPE) section prepared from the affected tissue can be used as a sample. When mRNA expression level is measured, a protein extract solution or mRNA extract solution prepared from the affected tissue can be used as a sample.

A known method that can be used is the immunohistochemical staining method, and as an example, the following method is exemplified. First, the affected tissue collected from the patient is fixed with formalin. The formalin-fixed tissue is embedded in paraffin, sliced into a thickness of about 3 to 4 µm with a microtome, and attached on a slide glass to prepare a section sample. The section sample is treated with xylene, then passed through an alcohol solution and returned to water to be deparaffinized. Thereafter, in order to enhance permeability of the antibody, the section sample is immersed in a citric acid buffer of pH 6.0 and heat-treated at 121°C for 10 minutes in an autoclave to activate an antigen. After deactivating endogenous peroxidase, an anti-DCBLD2 antibody is added dropwise to the section sample and allowed to react at room temperature for 1 hour. After washing, they are reacted with labeled antibodies, amplification reagents and the like, respectively. After washing, color development is carried out using DAB solution (3,3'-diaminobenzidine tetrahydrochloride). After washing with running water, cell nuclei of the sample are stained with hematoxylin solution. After rinsing with running water, the cell nuclei are dehydrated by passing through an alcohol solution followed by a xylene solution, an encapsulant is dropped onto the sample, a cover glass is covered thereover, and color development of DCBLD is observed with a microscope.

In the particular embodiments, when the measured expression level of DCBLD2 is greater than or equal to a predetermined threshold value, the myxofibrosarcoma of the patient can be determined to be invasive. On the other hand, when the measured expression level of DCBLD2 is less than the predetermined threshold value, the myxofibrosarcoma of the patient can be determined to be non-invasive.

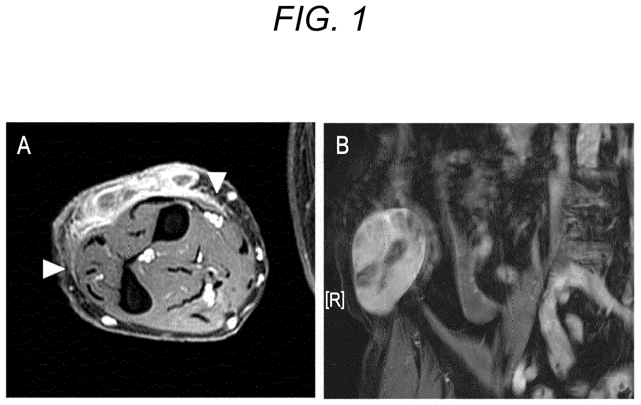

The predetermined threshold value is not particularly limited, and can be set empirically by accumulating data on various biological samples. For example, firstly, the affected tissues are classified into affected tissue evaluated as invasive and affected tissue evaluated as non-invasive, based on the MRI image. Next, the DBCLD2 expression level in each type of affected tissue is measured, and based on the result, a value that can distinguish both with high accuracy can be set as a threshold value. The threshold can be set in consideration of sensitivity, specificity, positive predictive value, negative predictive value, and the like. In the evaluation of invasiveness based on the MRI image, for example, a tumor tissue can be determined to be invasive when the presence of a tail like pattern is observed on the periphery of the tumor tissue in the MRI image, and a tumor tissue can be determined to be non-invasive when such pattern is not observed (see Fig. 1).

The threshold value may be provided stepwise. For example, the staining intensity by the immunohistochemical staining method can be evaluated at four levels of G0 to G3 according to the following criteria. Among them, the case of G2 or more (G2 to G3) can be determined to be invasive, and the case of less than G2 (G0 to G1) can be determined to be non-invasive.

### (Criteria)

G3: The entire cell membrane of the tumor cells is strongly stained.
G2: The entire cell membrane of the tumor cells is weakly to moderately stained.
G1: Faint/barely staining is observed in the cell membrane of the tumor cells.
G0: The tumor cells are not stained.

The degree of invasiveness of the myxofibrosarcoma can be determined based on the expression level of DCBLD2. For example, when the measured expression level of DCBLD2 is greater than or equal to than the predetermined threshold value, the degree of invasiveness of the myxofibrosarcoma of the patient can be determined to be high. In contrast, when the measured expression level of DCBLD2 is less than the predetermined threshold value, the invasiveness of the myxofibrosarcoma of the patient can be determined to be low.

The predetermined threshold value is not particularly limited, and can be set empirically by accumulating data on various biological samples. For example, firstly, a resection range is set apart from a tumor boundary specified based on the MRI image by a predetermined distance, and after resection, the affected tissues are histologically classified into an affected tissue evaluated as margin negative and an affected tissue evaluated as margin positive. Next, the DBCLD2 expression level in each affected tissue is measured, and based on the result, a value that can distinguish both with high accuracy can be set as a threshold value. The threshold can be set in consideration of sensitivity, specificity, positive predictive value, negative predictive value, and the like. For the determination of positive/negative of histological margin, for example, when the surface of a tumor resected specimen resected including a normal tissue on the MRI image apart from a tumor boundary based on the MRI image by a predetermined distance is evaluated to be histologically all normal tissues, the histological margin can be determined as negative, and when at least a part of the surface is not evaluated to be a normal tissue, the histological margin can be determined as positive. Histological evaluation can be performed based on, for example, the morphology of cells and tissues, continuity from a tumor and the like. The cut surface to be subjected to histological evaluation may include two cut surfaces, such asa cut surface including the major axis of the tumor (maximum cut surface) and a cut surface perpendicular thereto.

The scope of the present disclosure also includes use of a kit in the method of the invention. In a particular embodiment of the kit, an anti-DCBLD2 antibody can be contained in a reagent. Using the kit of the present invention, the expression level of DCBLD2 can be measured, for example, by immunological techniques such as immunohistochemical staining and Western blotting. The anti-DCBLD2 antibody is not particularly limited as long as it is an antibody capable of detecting the expression of DCBLD2. Examples of the anti-DCBLD2 antibody include monoclonal antibodies, polyclonal antibodies, labeled antibodies, chimeric antibodies, humanized antibodies and binding active fragments thereof, and the like. The kit of the present invention may contain reagents such as a secondary antibody and a blocking agent, in addition to the above antibody as well as reagents which are of particular use for the detection method envisaged, such as immunohistochemical staining reagents.

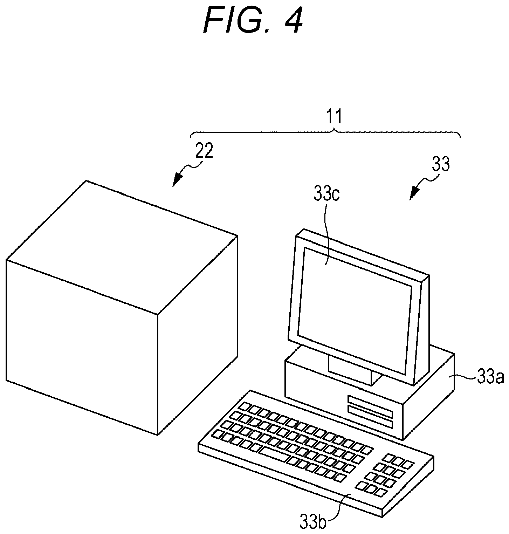

The invasiveness of myxofibrosarcoma can be determined, for example, by the determination apparatus 11 shown in Fig. 4. Hereinafter, a determination apparatus that can be used for determining the invasiveness of myxofibrosarcoma will be described in more detail with reference to the accompanying drawings, but the present disclosure is not limited to only this embodiment. Fig. 4 is a schematic explanatory view of an apparatus for determining the invasiveness of myxofibrosarcoma according to an embodiment of the present disclosure. The determination apparatus 11 shown in Fig. 4 includes a measurement device 22 and a computer system 33 connected to the measurement device 22.

In this embodiment, the measurement device 22 may include a scanner that detects signals from antibodies bound to DCBLD2 by immunohistochemical staining. In the present embodiment, the signal may be optical information on the expression level of DCBLD2 such as a fluorescent signal. The obtained optical information is transmitted to the computer system 33.

The scanner only needs to be capable of detecting a signal based on DCBLD2. Since the signal based on DCBLD2 differs depending on the labeling substance, the scanner can appropriately select one suitable for detecting a signal generated from the labeling substance, depending on the type of the labeling substance. For example, when the labeling substance is fluorescent, a scanner capable of detecting the fluorescence can be used as the measurement device 22. The optical information detected by the scanner is transmitted to the computer system 33.

The computer system 33 includes a computer main unit 33a, an input device 33b, and a display unit 33c that displays sample information, determination results, and the like. The computer system 33 receives the optical information from the measurement device 22. Then, the processor of the computer system 33 executes a program for determining the invasiveness of myxofibrosarcoma based on the optical information.

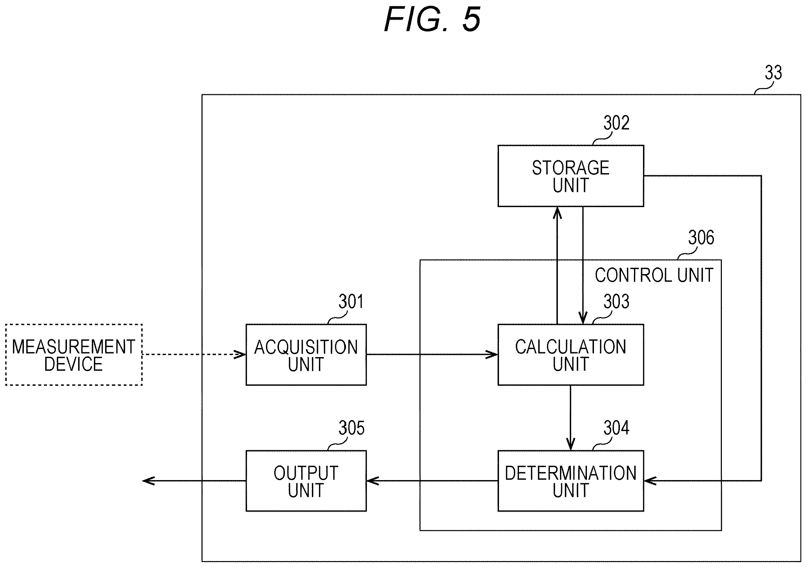

Fig. 5 is a block diagram showing a functional configuration of the determination assisting apparatus shown in Fig. 4.

As shown in Fig. 5, the computer system 33 includes an acquisition unit 301, a storage unit 302, a calculation unit 303, a determination unit 304, and an output unit 305. The acquisition unit 301 is communicably connected to the measurement device 22 via a network. The calculation unit 303 and the determination unit 304 constitute a control unit 306.

The acquisition unit 301 acquires the information transmitted from the measurement device 22. The storage unit 302 stores a threshold necessary for determination, an equation for calculating DCBLD2 expression level, a processing program for invasiveness determination, and the like. Using the information acquired by the acquisition unit 301, the calculation unit 303 calculates the DCBLD2 expression level using an equation, a processing program and the like stored in the storage unit 302. The determination unit 304 determines invasiveness, based on the value calculated by the calculation unit 303. The output unit 305 outputs the determination result of the determination unit 304 to the display unit 33c as the applicability of the invasiveness of myxofibrosarcoma.

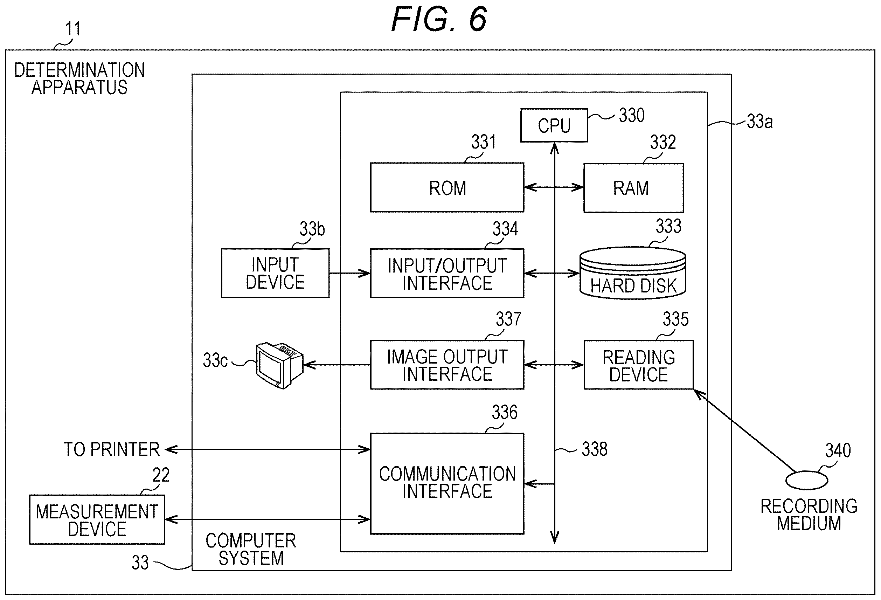

Fig. 6 is a block diagram showing a hardware configuration of the determination apparatus shown in Fig. 4.

As shown in Fig. 6, the computer main body 33a includes a CPU (Central Processing Unit) 330, a ROM (Read Only Memory) 331, a RAM 332, a hard disk 333, an input/output interface 334, a reading device 335, a communication interface 336, and an image output interface 337. The CPU 330, the ROM 331, the RAM (Random Access Memory) 332, the hard disk 333, the input/output interface 334, the reading device 335, the communication interface 336 and the image output interface 337 are data-communicably connected by a bus 338.

The CPU 330 can execute a computer program stored in the ROM 331 and a computer program loaded in the RAM 332. Each of the above-described functional blocks is realized by the CPU 330 executing an application program.

Thus, the computer system functions as a terminal of the apparatus for assisting determination of the invasiveness of myxofibrosarcoma.

The ROM 331 is constituted of a mask ROM, a PROM, an EPROM, an EEPROM, and the like. In the ROM 331, a computer program executed by the CPU 330 and data used for the computer program are recorded.

The RAM 332 is constituted of an SRAM, a DRAM, and the like. The RAM 332 is used for reading the computer program recorded in the ROM 331 and the hard disk 333. The RAM 332 is also used as a work area of the CPU 330 when executing these computer programs.

The hard disk 333 has installed therein an operating system to be executed by the CPU 330, a computer program such as an application program (a computer program for determination of the invasiveness of myxofibrosarcoma), and data used for executing the computer program.

The reading device 335 is constituted of a flexible disk drive, a CD-ROM drive, a DVD-ROM drive, and the like. The reading device 335 can read a computer program or data recorded on a portable recording medium 340.

The input/output interface 334 is constituted of, for example, a serial interface such as USB, IEEE1394 or RS-232C, a parallel interface such as SCSI, IDE or IEEE1284, and an analog interface including a D/A converter, an A/D converter and the like. The input device 33b such as a keyboard and a mouse is connected to the input/output interface 334. The operator can input data to the computer main unit 33a by using the input device 33b.

The communication interface 336 is, for example, an Ethernet (registered trademark) interface or the like. The computer system 33 can transmit print data to a printer through the communication interface 336.

The image output interface 337 is connected to the display unit 33c constituted of an LCD, a CRT, and the like. Accordingly, the display unit 33c can output the video signal corresponding to the image data given from the CPU 330. The display unit 33c displays an image (screen) according to the inputted video signal.

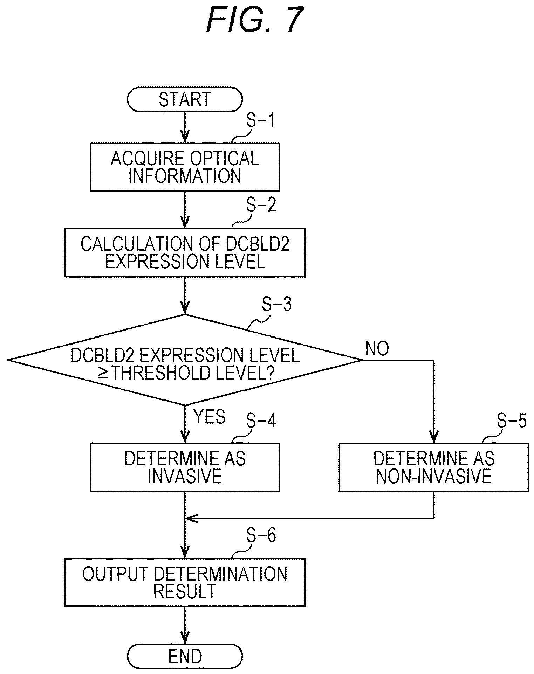

Next, the processing procedure for determining the invasiveness of myxofibrosarcoma by the determination apparatus 11 will be described. Fig. 7 is a flowchart of determination of the invasiveness of myxofibrosarcoma using the determination apparatus shown in Fig. 4. Here, the case where the determination is made using the fluorescence information based on the antibody bound to DCBLD2 of the section sample prepared from the affected tissue of the subject will be described as an example, but the present disclosure is not limited to only this embodiment.

First, in step S-1, the acquisition unit 301 acquires fluorescence information from the measurement device 22. In step S-2, the calculation unit 303 calculates the DCBLD2 expression level from the fluorescence information acquired by the acquisition unit 301. The calculation unit 303 transmits the DCBLD2 expression level to the storage unit 302.

Next, in step S-3, the calculation unit 303 determines whether or not the DCBLD2 expression level acquired in the storage unit 302 is greater than or equal to the threshold value stored in the storage unit 302. When the DCBLD2 expression level is greater than or equal to the threshold value, the process proceeds to step S-4. Then, the determination unit 304 transmits a determination result indicating that the myxofibrosarcoma is invasive to the output unit 305. On the other hand, when the DCBLD2 expression level is less than the threshold value, the process proceeds to step S-5. Then, the determination unit 304 transmits a determination result indicating that the myxofibrosarcoma is non-invasive to the output unit 305.

Thereafter, in step S-6, the output unit 305 outputs the determination result, the output unit 305 displays the determination result on the display unit 33c, and the output unit 305 makes a printer to print the determination result. Thus, a doctor or the like can provide information to assist in determining whether myxofibrosarcoma is invasive or non-invasive.

### EXAMPLES

Hereinafter, the present invention will be described in detail by examples, but the present invention is not limited to these examples.

### Example 1: Analysis of proteins by two-dimensional fluorescence difference gel electrophoresis (2D-DIGE)

(1) Resected specimens of 11 patients with myxofibrosarcoma were used (Table 1). Invasiveness was evaluated from MRI images of tumor tissues of each specimen and grouped into invasive group and non-invasive group. Invasiveness was evaluated by whether or not a tail like pattern was observed on the periphery of the tumor tissue in the MRI image (see Fig. 1). When a tail like pattern was observed, it was determined to be invasive, and when it was not observed, it was determined to be non-invasive.

**[Table 1]**

| Case No. | Sex | Age | Part | MRI |
|---|---|---|---|---|
| 1 | M | 70 | Limbs | Invasive |
| 2 | F | 29 | Limbs | Non-invasive |
| 3 | F | 48 | Limbs | Non-invasive |
| 4 | M | 70 | Limbs | Non-invasive |
| 5 | F | 67 | Limbs | Invasive |
| 6 | F | 66 | Trunk | Invasive |
| 7 | F | 63 | Limbs | Non-invasive |
| 8 | M | 66 | Trunk | Invasive |
| 9 | F | 62 | Limbs | Non-invasive |
| 10 | M | 72 | Limbs | Invasive |
| 11 | F | 71 | Limbs | Invasive |

### (2) Sample preparation

Frozen resected specimens were pulverized into powder form with liquid nitrogen using a multi-beads shocker (Yasui Kikai Corporation). The powder was treated with urea lysis buffer (6 mol/L urea, 2 mol/L thiourea, 3% CHAPS, 1% Triton X-100). After centrifugation at 15,000 rpm for 30 minutes, the supernatant was recovered and used as a protein sample. (3) All protein samples were mixed little by little in equal amounts to prepare an internal standard sample. The internal standard sample was labeled with Cy3, and each sample was labeled with Cy5 (both CyDye DIGE Fluor saturation dye, GE Healthcare Biosciences, Uppsala, Sweden). Samples labeled with these different fluorescent dyes were mixed and subjected to two-dimensional electrophoresis.

The first separation used a 24 cm long Immobilien gel (IPG, pI 4-7, GE Healthcare Biosciences) and Multiphor IITM (GE Healthcare Biosciences), and the second separation used a gradient gel prepared by GiantGelRunner (Biocraft, Tokyo, Japan) After electrophoresis, the gel was scanned using a laser scanner (Typhoon Trio, GE Healthcare Biosciences). In order to remove the difference between the gels, Cy5 intensity was corrected with Cy3 intensity of the same spot by software Progenesis SameSpots (Nonlinear Dynamics, Newcastle, UK). Experiments were performed three times for all samples, and statistical analysis processing was performed using the average value of the corrected intensities.

The fluorescence intensity data of the corrected protein spot was subjected to Wilcoxon test using software Expressionist (GeneData, Basel, Switzerland). By comparing the protein expression profiles of invasive group and noninvasive group, among 3453 protein spots, 59 protein spots satisfying the criterion that the difference in mean values was twice or more, and p < 0.05 by the Wilcoxon test, in the comparison between the 2 groups, were selected. The 59 protein spots were analyzed by mass spectrometry.

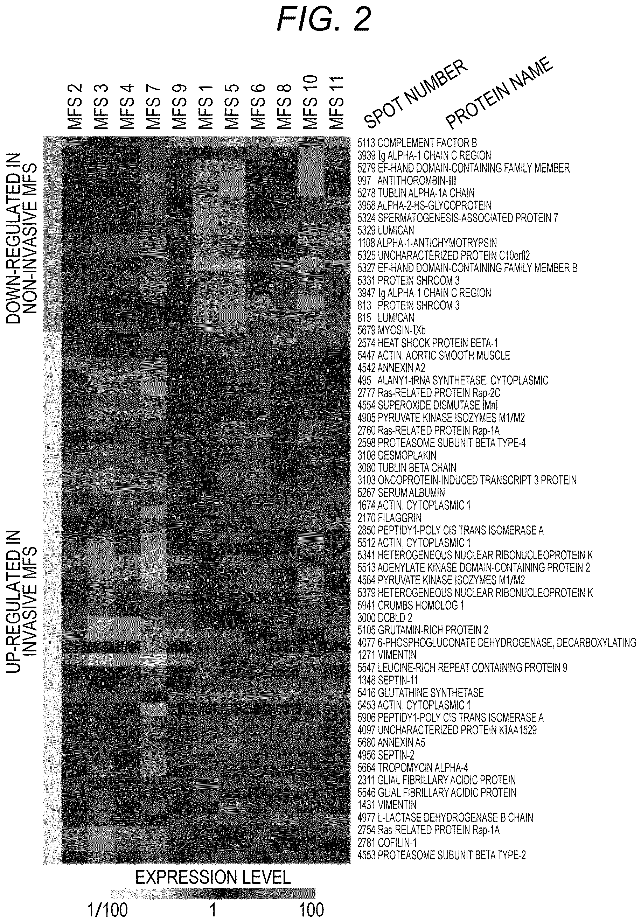

Each protein spot was recovered and digested with trypsin. The resulting trypsin digest was analyzed using a high performance liquid chromatograph tandem mass spectrometer equipped with a nanoion spray ion supply source (Finnigan LTQ linear ion trap mass spectrometer, Thermo Electron Co., San Jose, CA) to identify corresponding 47 proteins. The heat map is shown in Fig. 2. As a result of the verification experiment by immunostaining, DCBLD2 was specified as a protein especially highly related to invasiveness. Other proteins were not stained properly.

### Example 2: Expression verification by immunohistochemical staining

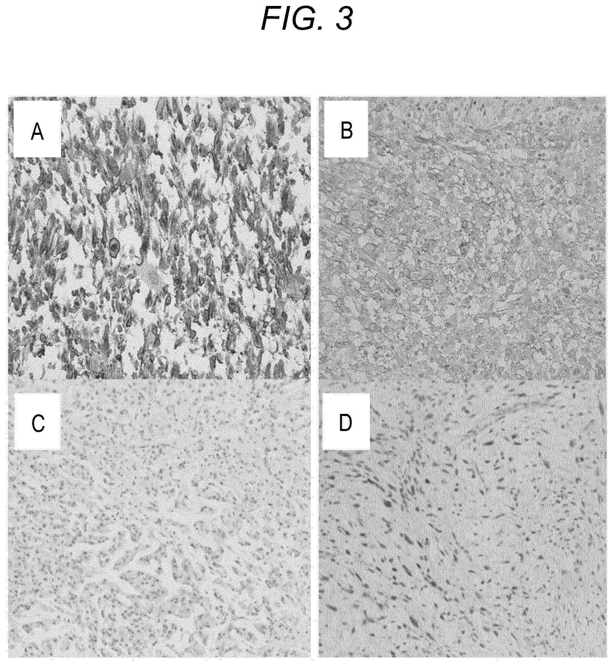

In order to verify the correlation between the DCBLD2 expression level and the invasiveness of myxofibrosarcoma, verification experiments were conducted on 21 new patients with myxofibrosarcoma. For all patients, gadolinium enhanced MRI imaging was performed to define the extent of the tumor region from gadolinium enhanced fat suppression T1-weighted images. The distal part 2 cm from the tumor margin specified based on the image was taken as the resection edge. Formalin-fixed paraffin-embedded tissue section samples were prepared from the resected specimens, and immunohistochemical staining was performed. For each section sample, both the central part and the peripheral part of the tumor tissue were examined. For immunohistochemical staining, the polymer method (Envision Dual Ling System-HRP, Dako, DK-2600, Glostrup, Denmark) was adopted. Section samples were deparaffinized and dehydrated, and then treated with 10 ml/l hydrogen peroxide-added methanol for 30 minutes to remove endogenous peroxidase activity. Antigen activation was performed by autoclaving at 121°C for 10 minutes in 10 mM citrate buffer (pH 6.0). The resulting section sample and the DCBLD2 antibody (Atlas Antibodies, Stockholm, Sweden) diluted 100-fold were allowed to react at room temperature for 1 hour. After reaction, color development was carried out using 3,3'-diaminobenzidine tetrahydrochloride. Thereafter, the reactant was counterstained with hematoxylin solution. The staining intensity was evaluated at four levels of G0 to G3 by two independently under blind according to the following criteria. G2-3 was classified as DCBLD2 positive and G0-1 as DCBLD2 negative. The results are also shown in Table 2. Photomicrographs of the tissues evaluated as G0 to G3 are shown in Fig. 3.

### (Criteria)

G3: The entire cell membrane of the tumor cells is strongly stained.
G2: The entire cell membrane of the tumor cells is weakly to moderately stained.
G1: Faint/barely staining is observed in the cell membrane of the tumor cells.
G0: The tumor cells are not stained.

Furthermore, after surgery, the positive/negative of histological margin was determined for all patients. For the determination of positive/negative of histological margin, when the surface of a tumor resected specimen resected including a normal tissue on the MRI image apart from a tumor boundary based on the MRI image by a distance of 2 cm was evaluated to be histologically all normal tissues, the histological margin was determined as negative, and when at least a part of the surface was not evaluated to be a normal tissue, the histological margin was determined as positive. The histological evaluation was performed based on the morphology of the cells and tissues continuity from a tumor and the like, on the cut surface including the major axis of the tumor (maximum cut surface) and the cut surface perpendicular thereto. The results are also shown in Table 2.

**[Table 2]**

| Case No. | Sex | Age | Part | MRI | Histological margin | DCBLD2 Staining intensity | | DCBLD2 Expression |
|---|---|---|---|---|---|---|---|---|
| | | | | | | Central part | Peripheral part | |
| 1 | F | 56 | Limbs | Non-invasive | - | G1 | G0 | Negative |
| 2 | F | 63 | Limbs | Invasive | - | G0 | G0 | Negative |
| 3 | M | 59 | Limbs | Non-invasive | - | G1 | G0 | Negative |
| 4 | F | 66 | Limbs | Invasive | - | G2 | G2 | Positive |
| 5 | F | 81 | Limbs | Invasive | + | G3 | G3 | Positive |
| 6 | M | 88 | Limbs | Non-invasive | - | G0 | G0 | Negative |
| 7 | F | 59 | Limbs | Invasive | + | G2 | G2 | Positive |
| 8 | M | 83 | Trunk | Non-invasive | - | G1 | G0 | Negative |
| 9 | F | 74 | Trunk | Invasive | - | G2 | G2 | Positive |
| 10 | M | 75 | Limbs | Non-invasive | - | G0 | G0 | Negative |
| 11 | M | 80 | Limbs | Invasive | + | G2 | G2 | Positive |
| 12 | M | 72 | Limbs | Invasive | - | G2 | G2 | Positive |
| 13 | F | 71 | Limbs | Invasive | - | G0 | G0 | Negative |
| 14 | F | 67 | Limbs | Non-invasive | - | G0 | G0 | Negative |
| 15 | M | 72 | Limbs | Non-invasive | - | G1 | G0 | Negative |
| 16 | F | 71 | Trunk | Non-invasive | - | G2 | G2 | Positive |
| 17 | F | 56 | Limbs | Invasive | - | G2 | G2 | Positive |
| 18 | F | 66 | Limbs | Invasive | - | G3 | G3 | Positive |
| 19 | M | 75 | Limbs | Invasive | - | G1 | G0 | Negative |
| 20 | M | 68 | Limbs | Invasive | - | G1 | G0 | Negative |
| 21 | M | 60 | Limbs | Invasive | + | G2 | G2 | Positive |

As an invasive biomarker of myxofibrosarcoma, the sensitivity and specificity of DCBLD2 in the central part of the tumor were 69.2% and 87.5%, respectively. The positive predictive value and the negative predictive value were 90% and 63.6%, respectively. The correlations between the DCBLD2 expression level and the invasiveness based on the MRI image and the DCBLD2 expression level and histological margin (positive/negative) were both statistically significant (P < 0.05) by sunivariate analysis. In all 10 cases that were DCBLD2 positive in the peripheral part of the tumor, DCBLD2 was also highly expressed in the central part.

In the treatment of myxofibrosarcoma, extensive resection that encapsulates normal tissue around the tumor tissue is necessary, in order to enhance the radicality of resection. Whether or not the tumor is invasive will determine the local recurrence rate and also influence the setting of the resection range in preoperative planning, so that it is important to determine invasiveness with high accuracy.

MRI, CT, PET and the like are used in diagnostic imaging of myxofibrosarcoma, but in particular, MRI is excellent in contrast resolution and can clearly depict a tumor region, thus the invasiveness of myxofibrosarcoma is generally determined based on the MRI image. A significant correlation was found between invasiveness determination based on the MRI image and the expression level of DCBLD2, and its sensitivity and specificity were 69.2% and 87.5%, respectively, and the positive predictive value and the negative predictive value were high as 90% and 63.6%, respectively. Therefore, the method for determining the invasiveness of myxofibrosarcoma using DCBLD2 as a biomarker can be a method complementing or replacing the determination based on the MRI image.

Histological evaluation of resection specimens after surgery may have invasion extending beyond the tumor boundary specified by the MRI image. For example, as a result of histological evaluation, when invasion has reached the margin or when the distance between the tip of invasion and the margin is not sufficiently secured, additional extensive resection or postoperative radiotherapy may be required in some cases, for preventing recurrence. The cause may be the presence of a minute tumor having a volume less than the volume of tissue that can be drawn in the MRI image.

In contrast, since a significant correlation was found between the DCBLD2 expression level and the histological margin, based on the DCBLD2 expression level measured by preoperative immunohistochemical staining or the like, whether the invasion extends beyond the tumor boundary drawn in the MRI image, and the degree of its invasiveness can be determined.

Further, in the conventional extensive resection of myxofibrosarcoma, the distance from the tumor boundary to the resection edge based on the MRI image was set at two levels depending on whether it is invasive or non-invasive, but it may be possible to set an appropriate resection range considering the recurrence prevention and the securing of the postoperative function, by further dividing the distance to the resection edge, according to the DCBLD2 expression level.

Even in determination of invasiveness/non-invasiveness, determination based on a conventional MRI image may be determined to be non-invasive when there is a minute invasive portion having a volume less than the volume of tissue that can be drawn. However, by using DCBLD2 that can also reflect such a minute tumor, as a biomarker, the accuracy of determination can be further improved.

## Claims

1. A method for assisting determination of the invasiveness of myxofibrosarcoma in a patient, comprising:
measuring the expression level of Discoidin, CUB and LCCL domain-containing protein 2 (DCBLD2) in a sample of affected tissue of said patient; and comparing said expression level of DCBLD2 measured in said sample of affected tissue of said patient with a predetermined threshold value, wherein said myxofibrosarcoma is determined to be invasive when said expression level of DCBLD2 is greater than or equal to a predetermined threshold value.

2. The method according to claim 1, wherein, in the determining step, when the expression level of DCBLD2 is less than the predetermined threshold value, myxofibrosarcoma of the patient is determined to be non-invasive.

3. The method according to claim 1 or 2, wherein the expression level of DCBLD2 is measured by immunohistochemical staining using an anti-DCBLD2 antibody.

4. The method according to any of claims 1 to 3, wherein the affected tissue of the patient comprises tumor cells.

5. Use of a kit in the method according to any of claims 1 to 4, the kit comprising a reagent capable of measuring the expression level of DCBLD2.

6. The use according to claim 5, wherein the reagent comprises an anti-DCBLD2 antibody.

## Patentansprüche

1. Verfahren zur Unterstützung der Feststellung der Invasivität von Myxofibrosarkom in einem Patienten, welches Folgendes umfasst:
das Messen des Expressionsniveaus von DCBLD2 (Discoidin, CUB and LCCL Domain-Containing Protein 2) in einer Probe von betroffenem Gewebe des Patienten und Vergleich des in der Probe von betroffenem Gewebe des Patienten gemessenen Expressionsniveaus von DCBLD2 mit einem vorbestimmten Schwellenwert, wobei festgestellt wird, dass das Myxofibrosarkom invasiv ist, wenn das Expressionsniveau von DCBLD2 größer als oder gleich einem vorbestimmten Schwellenwert ist.

2. Verfahren nach Anspruch 1, wobei, im Feststellungsschritt, wenn das Expressionsniveau von DCBLD2 geringer ist als der vorbestimmte Schwellenwert, festgestellt wird, dass das Myxofibrosarkom des Patienten nicht invasiv ist.

3. Verfahren nach Anspruch 1 oder 2, wobei das Expressionsniveau von DCBLD2 durch immunhistochemische Anfärbung mit einem Anti-DCBLD2-Antikörper gemessen wird.

4. Verfahren nach einem der Ansprüche 1 bis 3, wobei das betroffene Gewebe des Patienten Tumorzellen umfasst.

5. Verwendung eines Kits in dem Verfahren nach einem der Ansprüche 1 bis 4, wobei das Kit ein Reagenz umfasst, mit dem das Expressionsniveau von DCBLD2 gemessen werden kann.

6. Verwendung nach Anspruch 5, wobei das Reagenz einen Anti-DCBLD2-Antikörper umfasst.

## Revendications

1. Procédé d'aide à la détermination du pouvoir invasif d'un myxofibrosarcome chez un patient, comprenant les étapes consistant à :
mesurer le niveau d'expression de protéine 2 contenant des domaines discoïdine, CUB et LCCL (DCBLD2) dans un échantillon de tissu affecté dudit patient ; et comparer ledit niveau d'expression d'une DCBLD2 mesuré dans ledit échantillon de tissu affecté dudit patient avec une valeur seuil prédéterminée, dans lequel ledit myxo-fibrosarcome est déterminé comme étant invasif quand ledit niveau d'expression d'une DCBLD2 est supérieur ou égal à une valeur seuil prédéterminée.

2. Procédé selon la revendication 1, dans lequel dans l'étape de détermination, quand le niveau d'expression de DCBLD2 est inférieur à la valeur seuil prédéterminée, le myxo-fibrosarcome du patient est déterminé comme étant non invasif.

3. Procédé selon les revendications 1 ou 2, dans lequel le niveau d'expression d'une DCBLD2 est mesuré par coloration immunohistochimique en utilisant un anticorps anti-DCBLD2.

4. Procédé selon l'une quelconque des revendications 1 à 3, dans lequel le tissu affecté du patient comprend des cellules tumorales.

5. Utilisation d'une trousse dans le procédé selon l'une quelconque des revendications 1 à 4, la trousse comprenant un réactif capable de mesurer le niveau d'expression de DCBLD2.

6. Utilisation selon la revendication 5, dans laquelle le réactif comprend un anticorps anti-DCBLD2.
